# EUROPÄISCHE PATENTSCHRIFT

(11) **EP 1 429 709 B1**
(45) Veröffentlichungstag und Bekanntmachung des Hinweises auf die Patenterteilung: **01.08.2007**
(21) Anmeldenummer: 02785126.0
(22) Anmeldetag: 19.09.2002
(51) Int. Cl.: A61H 39/08, A61N 5/06

(54) **Vorrichtung zur Anbringung wenigstens einer Lasernadel am Körper eines Patienten**
Device for applying at least one laser needle on a patient's body
Dispositif d'application d'au moins une aiguille laser sur le corps d'un patient

(30) Priorität: 21.09.2001 DE 20115555 U
(43) Veröffentlichungstag der Anmeldung: 23.06.2004
(73) Patentinhaber: Weber, Michael, 37697 Lauenfoerde (DE)
(72) Erfinder: Weber, Michael, 37697 Lauenfoerde (DE)
(74) Vertreter: Seewald, Jürgen
(86) Internationale Anmeldenummer: PCT/EP2002/010498
(87) Internationale Veröffentlichungsnummer: WO 2003/026554

(56) Entgegenhaltungen:
- WO-A-99/62599
- DE-U- 20 019 703
- US-A- 3 735 753
- US-A- 5 643 173

## Beschreibung

Die Erfindung betrifft eine Vorrichtung zur Anbringung wenigstens einer Lasernadel am Körper eines Patienten.

Aus der DE 200 19 703 U1 ist eine Vorrichtung zur Akupunktur eines Patienten mittels Laserstrahlung bekannt. Zur Abringung am Körper des Patienten ist dabei ein Befestigungselement mit einem Klebering vorgesehen.

Für verschiedene Therapiearten ist ein solches Anbringen der Lasernadel über einen Klebering durchaus geeignet. Insbesondere wenn es sich jedoch um schwer zugängliche Körperteile oder um Körperteile handelt, an denen ein solcher Klebering keine ausreichende Haftung besitzen würde, sind andere Vorrichtungen erforderlich.

Eine derartige Vorrichtung ist aus der WO 99/62599 bekannt. Sie dient der Behandlung von örtlicher Blutleere im menschlichen Gehirn mit Laserstrahl und hat die Form eines Helms, der auf den Kopf aufgesetzt wird. In dem Helm ist eine Vielzahl von Laserquellen, z.B. Laserdioden integriert, von denen die Laserstrahlen über Lichtleitfasern zur Schädeldecke geleitet werden. Es sind Aktuatoren vorgesehen, mit denen die Laserquellen weiter oder näher zur Schädeldecke angeordnet werden können, wodurch der Abstand des distalen Endes der Lichtleitfasern zur Schädeldecke gesteuert werden kann. Mit dieser Vorrichtung ist eine flächige Bestrahlung eines menschlichen Schädels möglich, wobei die Einleitpunkte der Laserstrahlen in den Schädel nicht veränderbar sind, da diese durch die Anordnung der Laserstrahlenquellen in der Vorrichtung vorgegeben sind. Damit ist diese bekannte Vorrichtung für eine Laserakupunktur nicht geeignet, da eine wesentliche Voraussetzung für die Akupunktur die Stimulierung bestimmter Akupunkturpunkte ist. So können mit dieser Vorrichtung z.B. nicht Akupunkturpunkte im Bereich eines Ohres, im Halsbereich oder bestimmte Akupunkturpunkte im Schädelbereich erreicht werden.

Es ist daher Aufgabe der vorliegenden Erfindung, eine Vorrichtung zur Anbringung wenigstens einer Lasernadel am Körper eines Patienten zu schaffen, mit der die wenigstens eine Lasernadel sicher und fest am Körper des Patienten angebracht werden kann.

Erfindungsgemäß wird diese Aufgabe durch ein an ein Körperteil eines Patienten anpassbares Halteelement gelöst, an welchem wenigstens eine Führungseinrichtung angebracht ist, bestehend aus einem länglichen Führungselement aus plastisch verformbarem Draht, an dessen freiem Ende ein längliches Leitelement aus starrem Draht axial verschiebbar gelagert ist, welches in einer Aufnahme eine Lasernadel trägt.

Durch das erfindungsgemäße Halteelement und die wenigstens eine daran angebrachte Führungseinrichtung ist es vorteilhafterweise möglich, die wenigstens eine Lasernadel gleichzeitig fest und sicher und dabei dennoch verschieblich an einen gewünschten Körperteil des Patienten anzubringen.

Hierbei stellt das an den Körperteil des Patienten anpassbare Halteelement die feste Verbindung der Lasernadel mit dem Körperteil sicher, wohingegen die wenigstens eine Führungseinrichtung für die entsprechende Beweglichkeit der Lasernadel gegenüber dem Körperteil sorgt.

Da das längliche Leitelement aus einem plastisch verformbaren Draht besteht, lässt sich die Lasernadel sehr gut im Raum und somit gegenüber dem betreffenden Körperteil des Patienten orientieren. Dieses Orientieren der Lasernadel vereinfacht sich dadurch, dass an dem freien Ende der Führungseinrichtung ein längliches Leitelement aus starrem Draht axial verschiebbar gelagert ist, welches in einer Aufnahme die Lasernadel trägt.

In einer vorteilhaften Weiterbildung der Erfindung kann vorgesehen sein, dass an dem Halteelement mehrere Führungseinrichtungen zur Abringung mehrerer Lasernadeln angebracht sind.

Hierdurch lässt sich eine große Anzahl von Lasernadeln an dem jeweiligen Körperteil des Patienten anbringen, so dass, wie dies an sich in der klassischen Akupunktur gefordert wird, mehrere Stellen am Körper des Patienten gleichzeitig behandelt werden können.

Ein besonders stabiles Halteelement ergibt sich, wenn dasselbe in einer weiteren vorteilhaften Ausgestaltung der Erfindung eine dem Körperteil angepasste Schiene aufweist.

Um diese Schiene an dem Körperteil des Patienten anbringen zu können, kann des weiteren vorgesehen sein, dass die Schiene auf wenigstens einer Seite geöffnet und auf dieser Seite mittels wenigstens einer Verschließeinrichtung verschließbar ist, um ein ringförmiges Halteelement zu bilden.

Eine solche Verschließeinrichtung kann in konstruktiv einfacher Weise z.B. als Drehverschluß mit mehreren Rastpositionen oder als Klettverschluß ausgebildet sein, um eine einfache Handhabung derselben zu ermöglichen.

Als Alternative zu der Verschließeinrichtung kann die Schiene in einer weiteren Ausgestaltung der Erfindung auch derart federnd ausgebildet sein, dass sie mittels Federkraft an dem Körperteil klemmt. Auch hierdurch wäre eine einfache Bedienung der Vorrichtung sichergestellt.

Des weiteren kann vorgesehen sein, dass die Schiene auf ihrer dem Körperteil zugewandten Seite mit einem Band aus einem weichen Material versehen ist. Hierdurch ist ein Schutz des Körperteils, an dem die Schiene angebracht wird, gegeben.

Eine in der Praxis sehr einfach zu verwirklichende und für den täglichen Einsatz sehr gut verwendbare Möglichkeit zur Verbindung der wenigstens einen Führungseinrichtung mit dem Halteelement kann sich ergeben, wenn die wenigstens eine Führungseinrichtung über eine Hülse-Stecker-Verbindung mit dem Halteelement verbunden ist.

Hierbei kann vorgesehen sein, dass an dem Halteelement wenigstens eine der Anzahl der an dem Körperteil anzubringenden Lasernadeln entsprechende Anzahl an Hülse-Stecker-Verbindungen angebracht sind. Auf diese Weise können sämtliche Führungseinrichtungen identisch gefertigt werden und sind beliebig untereinander austauschbar.

Wenn an dem Führungselement der Stecker und an dem Halteelement die Hülsen der Hülse-Stecker-Verbindungen angeordnet sind, so läßt sich das Führungselement in besonders einfacher Weise an dem Halteelement befestigen.

Ein Schutz und zugleich eine. Stabilisierung für den plastisch verformbaren Draht kann sich ergeben, wenn der Draht über wenigstens annähernd seine gesamte Länge mit einem Schlauch überzogen ist.

In diesem Fall kann an dem Schlauch ein zweites Schlauchelement angebracht sein, durch welches das Leitelement durchgeschoben ist, um das Leitelement in besonders einfacher Weise mit dem Führungselement verbinden zu können und dennoch dessen Verschieblichkeit zu gewährleisten.

Das Leitelement aus starrem Draht ermöglicht es, durch Verschieben des Leitelementes die daran angebrachte Lasernadel sehr exakt gegenüber dem Körperteil des Patienten zu positionieren.

Zur Verbindung der Lasernadel mit dem Leitelement kann vorgesehen sein, dass an dem Leitelement eine Doppelhülse angebracht ist, welche die Aufnahme für die jeweilige Lasernadel aufweist.

Die erfindungsgemäße Vorrichtung läßt sich besonders gut einsetzen, wenn der Körperteil der Kopf des Patienten ist.

Im folgenden ist eine bevorzugte Ausführungsform der vorliegenden Erfindung prinzipmäßig dargestellt.

Es zeigt:
- Fig. 1: eine erfindungsgemäße Vorrichtung zur Anbringung mehrere Lasernadeln am Körper eines Patienten in einer Draufsicht; und
- Fig. 2: eine vergrößerte Darstellung einer der Führungseinrichtungen aus Fig. 1.

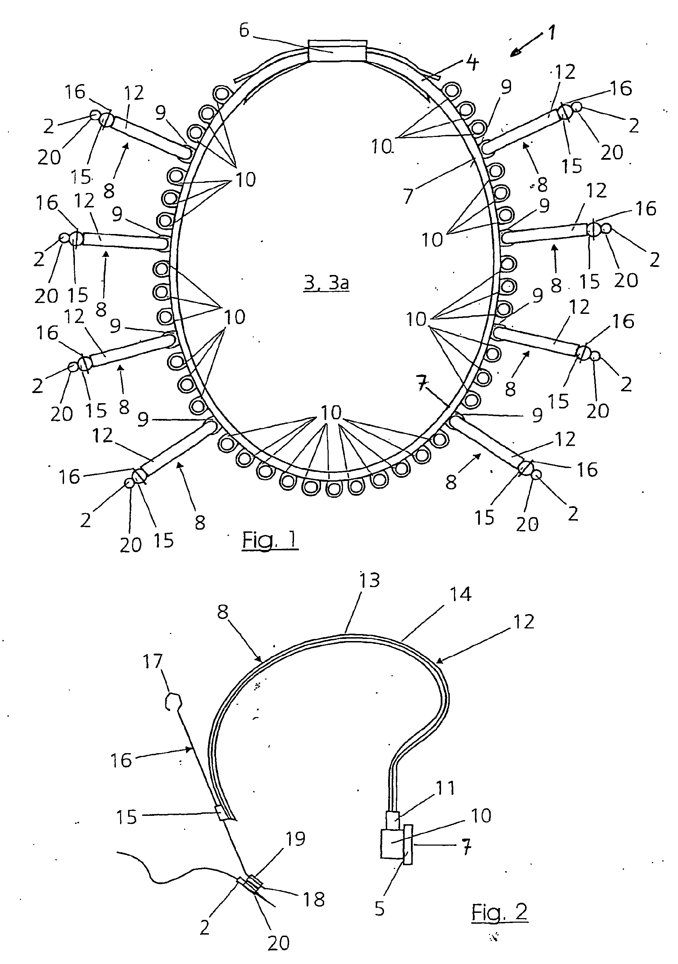

Fig. 1 zeigt eine Vorrichtung 1 zum Anbringen von im vorliegenden Fall acht einzelnen Lasernadeln 2 an einem Körperteil 3 eines Patienten, von dem nur der Körperteil 3 dargestellt ist. Mit den Lasernadeln 2 soll eine Akupunktur des Patienten durchgeführt werden. Bei dem Körperteil 3 handelt es sich im vorliegenden Fall um den Kopf 3a des Patienten an den die Vorrichtung 1 angepaßt ist. Es ist jedoch auch möglich, die Vorrichtung 1 an anderen Körperteilen, wie z.B. am Oberschenkel des Patienten, anzubringen oder die dargestellte Vorrichtung 1 derart umzugestalten, dass sie auch für andere Körperteile des Patienten geeignet sind. Des weiteren kann auch lediglich eine einzige oder jede beliebige andere Anzahl an Lasernadeln 2 an der Vorrichtung 1 angebracht werden.

Der Aufbau und die Funktionsweise der Lasernadeln 2 sind in der DE 200 19 703 U1 ausführlich beschrieben, weshalb im vorliegenden Fall nicht näher darauf eingegangen werden soll. Gegebenenfalls wäre es auch möglich, mit der Vorrichtung 1 andere, eventuell aus dem Stand der Technik bekannte Lasernadeln an dem Körperteil 3 des Patienten anzubringen.

Die Vorrichtung 1 weist ein Halteelement 4 auf, welches an das betreffende Körperteil 3 des Patienten angepaßt werden kann. Hierzu weist das Halteelement 4 im vorliegenden Fall eine dem Kopf 3a angepaßte Schiene 5 auf, die auf einer Seite, im vorliegenden Fall im hinteren Bereich des Kopfes 3a, geöffnet ist und dort mittels einer Verschließeinrichtung 6 verschließbar ist. Auf diese Weise bildet die Schiene 5 mit der Verschließeinrichtung 6 ein ringförmiges Halteelement 4, welches in seinem geschlossenen Zustand den gesamten Kopf 3a umschließt. Im geöffneten Zustand der Verschließeinrichtung 6 kann das Halteelement 4 dagegen sehr einfach an dem Kopf 3a angebracht werden.

Die Verschließeinrichtung 6 ist im vorliegenden Fall als Drehverschluß ausgebildet, der mehrere nicht näher dargestellte Rastpositionen aufweist, um das Halteelement 4 an verschiedene Kopfformen bzw. -größen anpassen zu können. Als Verschließeinrichtung 6 wäre beispielsweise auch ein Klettverschluß oder dergleichen denkbar. Des weiteren wäre auch vorstellbar, dass die Schiene 5 derart federnd ausgebildet ist, dass sie mittels Federkraft an dem Körperteil 3 klemmt und so ohne die Verschließeinrichtung 6 auskommt. Im vorliegenden Fall besteht die Schiene 5 aus Aluminium, wobei es selbstverständlich auch denkbar ist, dieselbe aus einem anderen Metall oder auch aus einem geeigneten Kunststoff herzustellen.

Auf ihrer dem Körperteil 3 zugewandten Seite ist die Schiene 5 mit einem Band 7 versehen, das aus einem weichen Material, wie z.B. Schaumstoff, besteht und so den jeweiligen Körperteil 3 vor dem Material der Schiene 5 schützt und auf diese Weise eventuellen Verletzungen vorbeugt. Bei der Verwendung der Vorrichtung 1 am Kopf 3a kann durch das Band 7 darüber hinaus auch eine erhöhte Rutschsicherheit erreicht werden, insbesondere in behaarten Bereichen.

Alternativ zu der Ausführung als umlaufende Schiene 5 könnte das Halteelement 4 auch mehrere einzelne Schienen 5 aufweisen, die beispielsweise durch das Band 7 zusammengehalten werden könnten.

An dem Halteelement 4 sind mehrere Führungseinrichtungen 8 zur Anbringung jeweils einer Lasernadel 2 angebracht. Im vorliegenden Fall weist die Vorrichtung 1 insgesamt acht Führungseinrichtungen 8 auf, an denen jeweils eine Lasernadel 2 angebracht werden kann, so dass mit der Vorrichtung 1 insgesamt acht Lasernadeln 2 an den Körperteil 3 des Patienten angebracht werden können.

Jede Führungseinrichtung 8 ist über eine jeweilige Hülse-Stecker-Verbindung 9 mit dem Halteelement 4 verbunden. Hierzu weist das Halteelement 4 im vorliegenden Fall insgesamt dreiundvierzig voneinander unabhängige Hülsen 10 auf, wohingegen jede einzelne Führungseinrichtung 8 einen mit jeder einzelnen Hülse 10 verbindbaren Stecker 11 aufweist. In nicht dargestellter Weise können die Stecker 11 selbstverständlich durch Schrauben oder andere Befestigungselemente an der jeweiligen Hülse 10 gesichert werden. Bei den Hülse-Stecker-Verbindungen 9 kann es sich beispielsweise um handelsübliche Lüsterklemmen handeln.

Einerseits wäre es auf diese Weise möglich, insgesamt dreiundvierzig der Führungseinrichtungen 8 an dem Halteelement 4 anzubringen. Andererseits, und dies ist der für die Praxis weitaus relevantere Fall, entsteht dadurch eine sehr große Kombinations- bzw. Variationsmöglichkeit, da die insgesamt acht Führungseinrichtungen 8 an den unterschiedlichsten Stellen am Umfang des Halteelements 4 angebracht werden können.

In einer nicht dargestellten Ausführungsform der Vorrichtung 1 wäre es auch möglich, die Hülsen 10 verschieblich an dem Halteelement 4 anzubringen, so dass gegebenenfalls nur eine der maximalen Anzahl an Lasernadeln 2 entsprechende Anzahl an Hülsen 10 an dem Halteelement 4 angebracht werden müßte, um eine entsprechende Positionierung zu ermöglichen.

Wie in Fig. 2 dargestellt, weist jede Führungseinrichtung 8 ein mit dem Halteelement 4 verbundenes, flexibles Führungselement 12 auf, an dem der Stecker 11 angebracht ist. Das Führungselement 12 wiederum weist einen aus einem plastisch verformbaren Material bestehenden Draht 13 auf, der vollständig mit einem Schlauch 14 überzogen ist. Der Draht 13 besteht im vorliegenden Fall aus Zinn, wohingegen der Schlauch 14, der zum Schutz und zur Stabilisierung des Drahtes 13 dient, aus Silikon besteht. Selbstverständlich sind für den Draht 13 und den Schlauch 14 auch andere Materialien denkbar, mit denen es möglich ist, das Führungselement 12 in eine bestimmte Position bzw. Form zu bringen, die dieses dauerhaft beibehält.

An dem Schlauch 14 ist ein zweites, sehr viel kürzeres Schlauchelement 15 angebracht, durch dessen Öffnung ein Leitelement 16 durchgeschoben ist. Im vorliegenden Fall ist das zweite Schlauchelement 15 einstückig mit dem Schlauch 14 ausgebildet, wobei es sich in der Praxis beispielsweise um ein zweiadriges Lautsprecherkabel handeln kann, das den Schlauch 14 und das Schlauchelement 15 beinhaltet.

Das Leitelement 16 ist als starrer Draht ausgebildet, der einen Handgriff 17 und ein im vorliegenden Fall um einen Winkel von ca. 20° abgebogenes Ende 18 aufweist, an dem eine Doppelhülse 19 angebracht ist. Die Doppelhülse 19 ist dabei ähnlich dem Bauteil Schlauch 14 / Schlauchteil 15 ausgebildet und weist eine schlauchförmige Aufnahme 20 für die jeweilige Lasernadel 2 auf.

Wenn die Lasernadel 2 in der Aufnahme 20 angebracht und das Führungselement 12 in eine geeignete Position gebracht wurde, dient das Leitelement 16 dazu, die Lasernadel 2 gegenüber dem Körperteil 3 zu verschieben und so in Kontakt mit demselben zu bringen. Hierzu kann das Leitelement 16 in der Aufnahme 20 sowohl verschoben als auch verdreht werden.

Auf diese Weise ist es also möglich, bis zu acht und mehr Lasernadeln 2 an dem Körperteil 3 anzubringen bzw. mit demselben in Berührung zu bringen und anschließend eine entsprechende Akupunkturbehandlung durchzuführen, ohne dass eine Vielzahl von Personen zum Halten der Lasernadeln 2 erforderlich sind.

Um für die Bedienperson der Vorrichtung 1 eine Kennzeichnung einzelner Führungseinrichtungen 8 zu schaffen, kann vorgesehen sein, dass die Führungselemente 12 und insbesondere deren jeweiliger Schlauch 14 mit einer bestimmten Farbe versehen ist.

Die Durchmesser des Schlauches 14, des Schlauchteils 15 und der Doppelhülse 19 sind so ausgelegt, dass die darin unterzubringenden Bauteile eine ausreichende Festigkeit und eine möglichst geringe Masse besitzen.

## Patentansprüche

1. Vorrichtung zur Anbringung wenigstens einer Lasernadel am Körper eines Patienten, aufweisend ein Halteelement (4) an welchem wenigstens eine Führungseinrichtung (8)angebracht ist, bestehend aus einem länglichen Führungselement (12) wobei diese Vorrichtung **dadurch gekennzeichnet ist, dass** das Halteelement (4) an ein Körperteil (3) des Patienten anpassbar ist, dass das längliche Führungselement (12) als plastisch verformbarer Draht (13) ausgebildet ist, und dass am freien Ende der Führungseinrichtung (8) ein längliches Leitelement (16) aus starrem Draht axial verschiebbar gelagert ist, welches eine Aufnahme (20) für die Lasernadel (2) aufweist.

2. Vorrichtung nach Anspruch 1, **dadurch gekennzeichnet, dass** an dem Haltelement (4) mehrere Führungseinrichtungen (8) zur Anbringung mehrerer Lasernadeln (2) angebracht sind.

3. Vorrichtung nach Anspruch 1 oder 2, **dadurch gekennzeichnet, dass** das Haltelement (4) eine dem Körperteil (3) angepasste Schiene (5) aufweist.

4. Vorrichtung nach Anspruch 3, **dadurch gekennzeichnet, dass** die Schiene (5) auf wenigstens einer Seite geöffnet und auf dieser Seite mittels wenigstens einer Verschließeinrichtung (6) verschließbar ist, um ein ringförmiges Halteelement (4) zu bilden.

5. Vorrichtung nach Anspruch 4, **dadurch gekennzeichnet**, das die Verschließeinrichtung (6) als Drehverschluss ausgebildet ist, der mehrere Rastpositionen aufweist.

6. Vorrichtung nach Anspruch 4, **dadurch gekennzeichnet, dass** die Verschließeinrichtung (6) als Klettverschluss ausgebildet ist.

7. Vorrichtung nach Anspruch 3, **dadurch gekennzeichnet, dass** die Schiene (5) derart federnd ausgebildet ist, dass sie mittels Federkraft an dem Körperteil (3) klemmt.

8. Vorrichtung nach einem der Ansprüche 3 bis 7, **dadurch gekennzeichnet, dass** die Schiene (5) auf ihrer dem Körperteil (3) zugewandten Seite mit einem Band (7) aus einem weichen Material versehen ist.

9. Vorrichtung nach einem der Ansprüche 1 bis 8, **dadurch gekennzeichnet, dass** die wenigstens eine Führungseinrichtung (8) über eine Hülse-Stecker-Verbindung (9) mit dem Halteelement (4) verbunden ist.

10. Vorrichtung nach Anspruch 9, **dadurch gekennzeichnet, dass** an dem Führungselement (12) der Stecker (11) und an dem Halteelement (4) die Hülsen (10) der Hülse-Stecker-Verbindungen (9) angeordnet sind.

11. Vorrichtung nach Anspruch 1, **dadurch gekennzeichnet, dass** der Draht (13) über wenigstens annähernd seine gesamte Länge mit einem Schlauch (14) überzogen ist.

12. Vorrichtung nach Anspruch 11, **dadurch gekennzeichnet, dass** an dem Schlauch (14) ein zweites Schlauchelement (15) angebracht ist, durch welches das Leitelement (16) durchgeschoben ist.

13. Vorrichtung nach einem der vorstehenden Ansprüche, **dadurch gekennzeichnet, dass** an dem Leitelement (16) eine Doppelhülse (19) angebracht ist, welche die Aufnahme (20) für die jeweilige Lasernadel (2) aufweist.

14. Vorrichtung nach einem der vorstehenden Ansprüche, **dadurch gekennzeichnet, dass** der Körperteil (3) der Kopf des Patienten ist.

## Claims

1. Apparatus for applying at least one laser needle to the body of a patient, comprising a retaining element (4), to which at least one guide device (8) is applied, this consisting of an elongate guide element (12), wherein this apparatus is **characterised in that** the retaining element (4) can be adapted to a body part (3) of the patient, that the elongate guide element (12) is formed as a plastically deformable wire (13), and that an elongate leading element (16) of rigid wire is mounted so as to be axially displaceable at the free end of the guide device (8) and comprises a receptacle (20) for the laser needle (2).

2. Apparatus according to Claim 1, **characterised in that** a plurality of guide devices (8) for applying a plurality of laser needles (2) are applied to the retaining element (4).

3. Apparatus according to Claim 1 or 2, **characterised in that** the retaining element (4) comprises a rail (5) which is adapted to the body part (3).

4. Apparatus according to Claim 3, **characterised in that** the rail (5) is open on at least one side and can be closed on this side by means of at least one closing device (6) in order to form an annular retaining element (4).

5. Apparatus according to Claim 4, **characterised in that** the closing device (6) is formed as a rotary closure which has a plurality of locking positions.

6. Apparatus according to Claim 4, **characterised in that** the closing device (6) is formed as a Velcro closure.

7. Apparatus according to Claim 3, **characterised in that** the rail (5) is formed in a resilient manner such that it clamps onto the body part (3) by means of spring force.

8. Apparatus according to any one of Claims 3 to 7, **characterised in that** the rail (5) is provided with a band (7) of a soft material on its side which faces the body part (3).

9. Apparatus according to any one of Claims 1 to 8, **characterised in that** the at least one guide device (8) is connected to the retaining element (4) via a sleeve-plug connection (9).

10. Apparatus according to Claim 9, **characterised in that** the plug (11) of the sleeve-plug connections (9) is disposed at the guide element (12) and the sleeves (10) of the sleeve-plug connections (9) are disposed at the retaining element (4).

11. Apparatus according to Claim 1, **characterised in that** the wire (13) is covered with a flexible tube (14) over at least approximately its entire length.

12. Apparatus according to Claim 11, **characterised in that** a second flexible tube element (15) is applied to the flexible tube (14), through which element the leading element (16) is pushed.

13. Apparatus according to any one of the preceding Claims, **characterised in that** a double sleeve (19), which comprises the receptacle (20) for the respective laser needle (2), is applied to the leading element (16).

14. Apparatus according to any one of the preceding Claims, **characterised in that** the body part (3) is the head of the patient.

## Revendications

1. Dispositif servant à fixer au corps d'un patient une aiguille laser au moins et présentant un élément de retenue (4), sur lequel est monté un dispositif de guidage (8) au moins, lequel dispositif de guidage est constitué d'un élément de guidage allongé (12), ce dispositif étant **caractérisé en ce que** l'élément de retenue (4) peut être adapté à une partie du corps du patient, **en ce que** l'élément de guidage allongé (12) est un fil métallique (13) pouvant être déformé plastiquement et **en ce qu'**un élément directeur allongé (16) en fil métallique, qui présente un logement (20) pour l'aiguille laser (2), est logé de manière déplaçable axialement à l'extrémité libre du dispositif de guidage (8).

2. Dispositif suivant la revendication 1, **caractérisé en ce que** plusieurs dispositifs de guidage (8) servant à fixer plusieurs aiguilles laser (2) sont montés sur l'élément de retenue (4).

3. Dispositif suivant la revendication 1 ou 2, **caractérisé en ce que** l'élément de retenue (4) présente un rail (5) adapté à la partie du corps (3).

4. Dispositif suivant la revendication 3, **caractérisé en ce que** le rail (5) est ouvert sur un côté au moins et peut être refermé sur ce côté au moyen d'un dispositif de fermeture (6) au moins, afin de former un élément de retenue annulaire (4).

5. Dispositif suivant la revendication 4, **caractérisé en ce que** le dispositif de fermeture (6) est conçu comme verrouillage tournant présentant plusieurs positions d'enclenchement.

6. Dispositif suivant la revendication 4, **caractérisé en ce que** le dispositif de fermeture (6) est conçu comme fermeture Velcro.

7. Dispositif suivant la revendication 3, **caractérisé en ce que** le rail (5) est conçu élastique de telle manière qu'il soit bloqué contre la partie du corps (3) grâce à l'élasticité.

8. Dispositif suivant une des revendications 3 à 7, **caractérisé en ce que** sur son côté tourné vers la partie du corps (3), le rail (5) est pourvu d'une bande (7) en une matière douce.

9. Dispositif suivant une des revendications 1 à 8, **caractérisé en ce que** le dispositif de guidage (8), dont au moins un exemplaire est prévu, est relié à l'élément de retenue (4) par l'intermédiaire d'un assemblage par douille et élément enfichable (9).

10. Dispositif suivant la revendication 9, **caractérisé en ce que** l'élément enfichable (11) de l'assemblage par douille et élément enfichable est disposé sur l'élément de guidage (12) et les douilles (10) de l'assemblage par douille et élément enfichable sont disposées sur l'élément de retenue (4).

11. Dispositif suivant la revendication 1, **caractérisé en ce que** le fil métallique (13) est recouvert d'un tuyau (14) sur approximativement toute sa longueur.

12. Dispositif suivant la revendication 11, **caractérisé en ce que** sur le tuyau (14) est monté un second élément tuyau (15), à travers lequel est introduit l'élément directeur (16).

13. Dispositif suivant une des revendications précédentes, **caractérisé en ce que** sur l'élément directeur (16) est montée une double douille (19), qui présente le logement pour l'aiguille laser (2) respective.

14. Dispositif suivant une des revendications précédentes, **caractérisé en ce que** la partie du corps (3) est la tête du patient.
